(19) Europäisches Patentamt
European Patent Office
Office européen des brevets

(11) EP 4 497 431 A1

(12) # EUROPEAN PATENT APPLICATION

(43) Date of publication:
**29.01.2025 Bulletin 2025/05**

(21) Application number: **23188159.0**

(22) Date of filing: **27.07.2023**

(51) International Patent Classification (IPC):
***A61K 9/00*** *(2006.01)* ***A61K 9/08*** *(2006.01)*
***A61K 38/18*** *(2006.01)* ***A61K 47/02*** *(2006.01)*
***A61K 47/18*** *(2017.01)* ***A61K 47/26*** *(2006.01)*
***A61K 47/34*** *(2017.01)*

(52) Cooperative Patent Classification (CPC):
**A61K 9/0019; A61K 9/08; A61K 38/185; A61K 47/02; A61K 47/183; A61K 47/26; A61K 47/34**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Dompé farmaceutici S.p.A.**
**20122 Milano (IT)**

(72) Inventors:
- **MATTIOLI, Simone**
  **67100 L'Aquila (IT)**
- **DETTA, Nicola**
  **80131 Napoli (IT)**
- **APPARENTE, Lucia**
  **67100 L'Aquila (IT)**
- **ROMEO, Tiziana**
  **60100 L'Aquila (IT)**

(74) Representative: **Dompé farmaceutici Spa**
**Via San Martino, 12-12/a**
**20122 Milano (IT)**

Remarks:
The complete document including Reference Table(s) and the Sequence Listing(s) can be downloaded from the EPO website

(54) **PHARMACEUTICAL FORMULATION COMPRISING NERVE GROWTH FACTOR**

(57) A liquid pharmaceutical formulation, suitable for lyophilization, comprising nerve growth factor (NGF) and at least one pharmaceutically acceptable excipient.

EP 4 497 431 A1

## Description

### FIELD OF THE INVENTION

The present invention relates to a pharmaceutical formulation comprising nerve growth factor (NGF), and at least one pharmaceutically acceptable excipient.

### STATE OF THE ART

The nerve growth factor (NGF) is a protein and member of the family of evolutionarily well-conserved neurotrophin growth factors that are required for the development and survival of specific neuronal populations.

NGF exerts its activity by interacting with two structurally unrelated cell surface receptors: the high affinity receptor tyrosine kinase A (TrKA) and the low affinity p75 neurotrophin receptor ($p75^{NTR}$).

This protein regulates the survival, development and function of neurons, both at a central and peripheral level.

NGF has been suggested to be effective for treating certain degenerative diseases of both the peripheral and central nervous systems, including hereditary sensory and motor neuropathies, hereditary and sporadically occurring system degeneration, amyotrophic lateral sclerosis Parkinson's disease, and Alzheimer's disease. Nowadays, NGF also finds a specific therapeutic use in the treatment of corneal diseases such as neurotrophic keratitis. NGF currently used in therapy is extracted from mouse submaxillary glands or manufactured by recombinant technologies.

The use of NGF in therapy poses some problems as its bioactivity, which depends on its secondary and tertiary structure stability, may be altered during manufacturing, purification or storage.

In addition to problems of stability, like many other proteins, NGF binds non-specifically to surfaces and to a variety of materials, including glass and plastics (e.g. polyethylene or polypropylene), with which NGF may come in contact during manufacture, storage or administration. This phenomenon may lead to a reduction in the concentration of the active in the solution and, consequently, to variable or insufficient amounts of NGF finally administered to the patient.

In order to mitigate the adhesion of NGF to primary packaging, the inner contact surface of NGF currently marketed glass vials may be covered with a coating that reduces the adsorption of proteins.

Alternatively, or in combination with using covered glass vials, NGF-containing aqueous formulations may be prepared and subsequently frozen; the frozen formulations require storage at low temperatures, usually -20°C, to minimize NGF aqueous formulations degradation and adhesion.

However, when the frozen NGF-containing aqueous formulations have to be used or administered, it is necessary to thaw the frozen formulation; as well-known in the art, the thawing process of the frozen protein-containing aqueous formulations typically causes several damages to the structure of proteins, including for example recrystallization phenomena during slow thawing rates.

Freeze-drying, or lyophilization, is another well-known method for the preparation of protein formulations, in particular in form of a powder formulation.

However, proteins are usually surface active and vulnerable to aggregation due to surface-induced denaturation. Adsorption or binding can occur at various interfaces during the lyophilization process and reconstitution of the obtained freeze dried formulations, for example, at the air-liquid interface due to mixing of liquid formulation components and at ice-liquid or ice-air interfaces during freezing and thawing or drying.

In addition, during lyophilization the protein is exposed to a number of stresses that may cause denaturation, including cold denaturation, aggregation, an increase in interfaces due to ice crystal formation, an increase in solute concentration (cryoconcentration) that can drastically alter the ionic strength and pH and phase separations. Furthermore, reconstitution also accelerates water-mediated destabilization pathways.

Therefore, it is known in the art the addition of stabilizing excipient, such as cryoprotectants, desiccoprotectans and surfactants to protein formulations, in order to stabilize proteins included therein.

However, as clearly taught in the scientific review of Thakral S. et al., "Stabilizers and their interaction with formulation components in frozen and freeze-dried protein formulations", (Advanced Drug Delivery Reviews 173 (2021) 1-19), when a surfactant solution is cooled, the ice crystallization will cause pronounced surfactant concentration, thus resulting in surfactant micelle formation in the freeze concentrate.

To avoid the surfactant micelle formation, the above-mentioned review teaches to use surfactants, in particular polysorbates, at concentrations lower than 0.1% (w/v).

In the light of the above, there is still the need to provide a pharmaceutical formulation comprising NGF, which is able to guarantee a high recovery of the protein, thus limiting phenomena of adhesion of NGF to packaging contact surfaces, and/or degradation of NGF, during preparation, storage and handling steps of the pharmaceutical formulation.

## SUMMARY OF THE INVENTION

The present inventors have now surprisingly found a lyophilized pharmaceutical nerve growth factor (NGF) formulation which, when reconstituted in a suitable reconstitution vehicle, is characterized by an unexpected high recovery of NGF.

Accordingly, it is an object of the present invention to provide a liquid pharmaceutical formulation, suitable for lyophilization, comprising nerve growth factor (NGF), and at least one pharmaceutically acceptable excipient.

Another object of the present invention is a method for preparing a nerve growth factor (NGF) formulation in form of powder, comprising the step of: lyophilizing said liquid pharmaceutical formulation, to obtain said nerve growth factor (NGF) formulation in form of powder.

A further object of the invention is a pharmaceutical formulation comprising nerve growth factor (NGF), in form of powder, obtained by said method.

A further object of the invention is said formulations comprising nerve growth factor (NGF) for use in the treatment of a nerve injury.

## BRIEF DESCRIPTION OF THE FIGURES

Figure 1 reports the thermogram of a rhNGF freeze-dried formulation containing PEG6000, according to Example 4 of the present invention.

Figure 2 reports the thermogram of a rhNGF freeze-dried formulation containing Kolliphor P188 (Poloxamer 188), according to Example 4 of the present invention.

## DETAILED DESCRIPTION OF THE INVENTION

The present invention is based on the observation of the unexpected high recovery of nerve growth factor (NGF) comprised in the lyophilized pharmaceutical formulation according to the present invention, after reconstitution thereof in a suitable reconstitution vehicle. Accordingly, an object of the present invention is a liquid pharmaceutical formulation, suitable for lyophilization, comprising nerve growth factor (NGF), and at least one pharmaceutically acceptable excipient according to the present invention.

The terms "lyophilization" and "freeze-drying" are used interchangeably herein.

The terms "lyophilized" and "freeze-dried" are used interchangeably herein.

Preferably, said liquid pharmaceutical formulation, suitable for lyophilization, has a pH between 6.5 and 8, more preferably between 6.8 and 7.5, even more preferably between 7 and 7.4.

Preferably, said liquid pharmaceutical formulation suitable for lyophilization, has an osmolarity between 200 and 400 mOsmol/kg, more preferably between 250 and 350 mOsmol/kg, even more preferably equal to 300 mOsmol/kg.

Preferably, said nerve growth factor (NGF) is human nerve growth factor (hNGF). Preferably, said NGF has the amino acid sequence of SEQ. ID NO.1 below:
SEQ. ID NO.1:

SSSHPIFHRGEFSVCDSVSVWVGDKTTATDIKGKEVMVLGEVNINNSVFKQYFFETKCRD
PNPVDSGCRGIDSKHWNSYCTTTHTFVKALTMDGKQAAWRFIRIDTACVCVLSRKAVR

Alternatively, said NGF has the amino acid sequence of SEQ ID NO. 2 below:
SEQ ID NO. 2:

SSSHPIFHRGEFSVCDSVSVWVGDKTTATDIKGKEVMVLGEVNINNSVFKQYFFETKCRD
PNPVDSGCRGIDSKHWNSYCTTTHTFVKALTMDGKQAAWRFIRIDTACVCVLSRKAVRR
A

Alternatively, said NGF is a mixture of NGFs having sequences of SEQ ID NO. 1 and SEQ ID NO. 2.

Preferably, said NGF is a recombinant hNGF (rhNGF), produced by recombinant DNA technology. Methods of producing rhNGF are known to the person skilled in the art, for example those described in WO0022119A1 and WO2013092776A1.

Preferably, said NGF has a purity higher than 70% w/w, more preferably higher than 80% w/w, higher than 90% w/w, higher than 95% w/w, higher than 98% w/w or higher than 99% w/w. The purity of NGF may be determined by conventional means known to those skilled in the art, for example by HPLC analysis.

Preferably, said NGF, as described above, is present in said liquid pharmaceutical formulation, suitable for lyophilization, at a concentration between 5 μg/ml and 300 μg/ml, more preferably between 10 μg/ml and 50 μg/ml, even more preferably between 15 μg/ml and 25 μg/ml, most preferably of 20 μg/ml.

Preferably, said at least one pharmaceutically acceptable excipient is selected from pharmaceutically acceptable viscosity enhancers, penetration enhancers, buffering agents, osmolarity regulators, preservatives, antioxidants, surfactants, emulsifying agents, cryoprotectant agents and bulking agents.

Preferably, said pharmaceutical formulation, suitable for lyophilization, according to the invention comprises one or more excipients selected from at least one buffering agent, at least one antioxidant, at least one poloxamer (as surfactant), at least one cryoprotectant agent, and at least one bulking agent.

Preferably, said liquid pharmaceutical formulation, suitable for lyophilization, according to the present invention, comprises:

- said nerve growth factor at a concentration between 5 μg/ml and 300 μg/ml, preferably between 10 μg/ml and 50 μg/ml, more preferably between 15 μg/ml and 25 μg/ml, even more preferably of 20 μg/ml;
- said at least one cryoprotectant agent at a concentration between 30 mg/ml and 60 mg/ml, preferably between 40 mg/ml and 55 mg/ml;
- said at least one bulking agent at a concentration between 5 mg/ml and 20 mg/ml, preferably between 10 mg/ml and 15 mg/ml;
- said at least one antioxidant at a concentration between 0.005 mg/ml and 0.020 mg/ml, preferably between 0.008 mg/ml and 0.015 mg/ml, more preferably of 0.01 mg/ml;
- said at least one poloxamer at a concentration of at least 0.25 mg/ml, preferably between 1 mg/ml and 4 mg/ml;
- said at least one buffer agent in an amount sufficient to maintain the pH of the formulation comprised between 6.5 to 8, and
- water.

Preferably, said at least one bulking agent is selected from mannitol, sorbitol, inositol, dulcitol, xylitol, arabitol, or a mixture thereof.

Preferably, said at least one bulking agent is mannitol, more preferably D-mannitol, and it is present in said liquid pharmaceutical formulation at a concentration between 5 mg/ml and 20 mg/ml, more preferably between 10 mg/ml and 15 mg/ml.

Preferably, said at least one cryoprotectant agent is selected from fructose, glucose, mannose, sorbose, xylose, maltose, lactose, sucrose, dextran and trehalose, or a mixture thereof.

Preferably, said at least one cryoprotectant agent is trehalose, more preferably trehalose dihydrate, and it is present in said liquid pharmaceutical formulation at a concentration between 30 mg/ml and 60 mg/ml, more preferably between 40 mg/ml and 55 mg/ml. Preferably, said at least one antioxidant is selected from L-methionine and cysteine, preferably L-methionine, and it is present in said liquid pharmaceutical formulation at a concentration between 0.005 mg/ml and 0.020 mg/ml, more preferably between 0.008 mg/ml and 0.015 mg/ml, even more preferably of 0.01 mg/ml.

Preferably, said at least one poloxamer is Poloxamer 188 (Kolliphor P188), and it is present in said liquid pharmaceutical formulation at a concentration of at least 0.25 mg/ml, more preferably between 1 mg/ml and 4 mg/ml.

The term "Kolliphor P188", as used herein, refers to Poloxamer 188, a block copolymer that is a synthetic copolymer of ethylene oxide and propylene oxide represented by the following chemical structure:

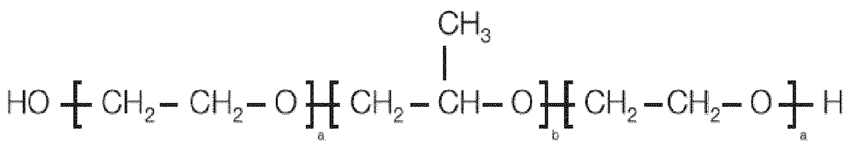

where in a and b blocks have the following values:

| Kolliphor® | Poloxamer | a | b |
|---|---|---|---|
| P 188 | 188 | 80 | 27 |

Preferably, said at least one buffering agent has a composition and concentration such as to obtain a pH of the

liquid pharmaceutical formulation between 6.5 and 8, more preferably between 6.8 and 7.5, even more preferably between 7 and 7.4. Preferably, said buffering agent is phosphate buffer and/or sodium hydroxide.

According to a preferred embodiment, said liquid pharmaceutical formulation of the invention comprises trehalose, mannitol, L-methionine, Poloxamer 188, and a buffer agent. Preferably, the liquid pharmaceutical formulation, according to the present invention, is an aqueous pharmaceutical formulation, suitable for lyophilization.

According to a preferred embodiment of the invention, said liquid pharmaceutical formulation, suitable for lyophilization, comprises:

- said nerve growth factor at a concentration between 10 μg/ml and 50 μg/ml, preferably between 15 μg/ml and 25 μg/ml, more preferably of 20 μg/ml;
- said trehalose at a concentration between 40 mg/ml and 55 mg/ml;
- said mannitol at a concentration between 10 mg/ml and 15 mg/ml;
- said L-methionine at a concentration between 0.008 mg/ml and 0.015 mg/ml;
- said Poloxamer 188 at a concentration between 1 mg/ml and 4 mg/ml;
- said buffer agent in an amount sufficient to maintain the pH of the formulation comprised between 6.5 to 8, and
- water.

The inventors have surprisingly found out that said concentration range of Poloxamer 188, as defined above, which is much higher than that taught in prior art (namely <0.1% w/v), allow to obtain an unexpected high recovery of NGF after reconstitution of the freeze-dried product in a suitable reconstitution solvent, at the same time advantageously preventing negative effects on the morphology of the freeze-dried pharmaceutical formulation cake. For example, a negative effect on the morphology of a freeze-dried cake may be the bilayer stratification of said cake.

Indeed, this concentration range of Poloxamer P188 according to the present invention advantageously guarantees the integrity of the lyophilized pharmaceutical formulation cake, with absence of any bilayer formation

Interestingly, a concentration higher than the concentration range of Poloxamer P188 according to the present invention causes several disadvantages on quality and/or biological activity of the reconstituted pharmaceutical formulation according to the present invention, including for example micelle formation in the freeze concentrate.

A concentration lower than the concentration range of Poloxamer P188 according to the present invention does not allow a high recovery of NGF, for example a recovery higher than 90% of NGF, thus determining a quality and/or biological activity decrease of the reconstituted pharmaceutical formulation of the present invention.

Moreover, Poloxamer 188 is conveniently able to decreases interfacial S/L forces during the freezing phase, while the water crystals are forming.

Furthermore, Poloxamer 188 advantageously doesn't re-crystalize during the drying step of the freezing-drying process, as clearly shown in Example 4; therefore, it doesn't cause any problems during drying step of freeze-drying.

As used herein, the expression "recovery of NGF" is the ratio between the concentration of rhNGF in the reconstituted sample at time n (where n is the stability time point) and the concentration of rhNGF in the reconstituted sample at time 0 (where time 0 is the initial stability time point).

According to this definition, it is particularly advantageous to obtain a recovery of NGF as high as possible in the reconstituted pharmaceutical formulation, thus minimizing the quantity of NGF adhered to the contact surface.

Advantageously, said concentration range of Poloxamer P188 comprised in the pharmaceutical formulation, according to the present invention, guarantees at least 90%, preferably at least 92.5%, more preferably at least 95%, most preferably 99%, advantageously 100% of NGF recovery in the reconstituted pharmaceutical formulation, as clearly shown in the Examples below.

The present invention further provides a method for preparing a pharmaceutical formulation in form of powder, preferably a lyophilized powder pharmaceutical formulation, comprising the step of: lyophilizing the above liquid pharmaceutical formulation, to obtain said pharmaceutical formulation in form of powder.

The present invention also provides a pharmaceutical formulation comprising nerve growth factor (NGF), in form of powder, obtained by said method.

Advantageously, the appearance of the lyophilized pharmaceutical formulation cake according to the present invention is completely white, and free from visible foreign matter.

The pharmaceutical formulation comprising nerve growth factor (NGF), in form of powder, according to the invention, is to be reconstituted with a suitable reconstitution vehicle, for example water for injection (WFI), before the administration to a subject.

Preferably, the reconstitution WFI volume is 1 mL or 2 mL WFI, thus obtaining a rhNGF concentration, in said reconstituted pharmaceutical formulation, equal to 10 μg/mL or 5 μg/mL, respectively.

The appearance of the thus-obtained reconstituted pharmaceutical formulation is advantageously free from visible particulate, and it appears clear and colourless, comparable to WFI.

Preferably, said reconstituted pharmaceutical formulation has an osmolarity between 70 and 200 mOsmol/kg,

more preferably between 75 and 150 mOsmol/kg.

More preferably, when said reconstitution WFI volume is 1 mL, the thus-obtained pharmaceutical formulation has an osmolarity of 150 mOsmol/kg; when said reconstitution WFI volume is 2 mL, the thus-obtained pharmaceutical formulation has an osmolarity of 75 mOsmol/kg.

Preferably, said reconstituted pharmaceutical formulation has a pH between 6.5 and 8, more preferably between 6.8 and 7.8, even more preferably between 7.2 and 7.6.

Preferably, said reconstituted pharmaceutical formulation has a moisture content (% w/w, determined by KF) comprised between 0.5% and 1.5%, preferably between 0.6% and 1%. Upon reconstitution, the reconstituted pharmaceutical formulation may be immediately administered to the subject.

The pharmaceutical formulations according to the invention are for use in the prevention or treatment of a nerve injury.

For the purposes of the present invention said pharmaceutical formulations according to the invention, preferably in the form of sterile solutions, are suitable for use by topical administration, parenteral administration, intravitreal administration, intravenous administration, or intramuscular administration.

Preferably, said pharmaceutical formulations according to the invention are for use in the treatment of an ocular disorder, said compositions being preferably administered topically to the eye.

Preferably, said ocular disorder is a corneal, conjunctival, scleral, retinal or optic nerve disorder.

More preferably said ocular disorder is selected from toxic and phototoxic keratopathy, iatrogenic, immune and neurotrophic corneal epithelial defects and ulcers, neurotrophic keratopathy, neuropathic corneal pain, keratoconjunctivitis sicca, Sjögren's syndrome-associated dry eye, retinitis pigmentosa, retinal detachment, retinal degeneration subsequent to ischemia, phototoxic retinopathy, epiretinal membrane, macular hole, macular degeneration, optic neuropathies, glaucoma, scleritis and episcleritis.

In any case, the regimen and amount of medicament to be administered will be determined by the physician according to the patient's need.

The invention will be now further illustrated in greater details in the following experimental section.

## EXPERIMENTAL PART

### Example 1 - Preparation of the pharmaceutical formulations according to the invention.

Weight and solubilize in the appropriate volume of WFI the following excipients:

| Excipient | Concentration (mg/mL) | Concentration (%) |
|---|---|---|
| Sodium Phosphate dibasic anhydrous | 3.065 | 0.3065 |
| Sodium Phosphate monobasic monohydrate | 1.16 | 0.116 |
| Kolliphor P188 | 4.00 | 0.4 |
| Trehalose dihydrate | 50.40 | 5.04 |
| D-Mannitol | 13.10 | 1.31 |
| L-Methionine | 0.0107 | 0.001 |
| 1.0N Sodium Hydroxide | q.s. to pH 7.20 ± 0.20 | q.s. to pH 7.20 ± 0.20 |
| Water for Injection PhEur/USP WFI | q.b. | q.b. |

Wait for the complete solubilization of excipients.

Add the rhNGF at the concentration of 0.020 mg/mL.

The thus-obtained formulation, after loading of rhNGF, is distributed in 2R glass vials (nominal volume of 0.5 mL), and then freeze-dried using VirTis AdVantage Pro Freeze Dryer (SP Scientific), according to the following lyophilization process:

| Description | Temperature (°C) | Pressure | Time (hh:min) |
|---|---|---|---|
| Load | +5 | Atmospheric | N.A. |
| Freezing | +5 → -50 | Atmospheric | 00:55 |
| Freezing | -50 | Atmospheric | 02:00 |

(continued)

| Description | Temperature (°C) | Pressure | Time (hh:min) |
|---|---|---|---|
| Evacuation | -50 | 100 μbar | 00:10 |
| Primary drying | -50 → -35 | 100 μbar | 00:15 |
| Primary drying | -35 | 100 μbar | 20:00 |
| Primary drying | -35-> -15 | 100 μbar | 00:50 |
| Primary drying | -15 | 100 μbar | 04:00 |
| Secondary drying | -15 → +35 | 100 μbar | 03:20 |
| Secondary drying | +35 | 100 μbar | 12:05 |
| Secondary drying | +35 → +5 | 100 μbar | 00:30 |
| Storage | +5 | 700 ± 10 mbar | N.A. |
| Pre-aeration with nitrogen (Backfill) | +5 | 700 ± 10 mbar | N.A. |
| Stoppering | +5 | 700 ± 10 mbar | N.A. |
| Aeration with nitrogen | +5 | Athmospheric | N.A. |
| Crimping | RT | Athmospheric | N.A. |
| | **Total Time Length (without Stoppering):** | | **44:05** |

Each of the two vials therefore contains a freeze-dried formulation comprising 0.010 mg rh NGF, and 2 mg Kolliphor.

To be administered, one of the freeze-dried formulations is reconstituted in 2 mL WFI, thus obtaining a reconstituted pharmaceutical formulation comprising 0.005 mg/ml (5 μg/mL) rhNGF and 1 mg/ml Kolliphor P188 (0.1%).

**Example 2** - **Effects of different concentrations of Poloxamer 188 on NGF recovery**

In order to compare the effects of different concentrations of Poloxamer 188 on NGF recovery, each the following liquid formulations was prepared into a vial made of double silicate glass, at room temperature:

Formulation A: 0.005mg/mL rhNGF in placebo + Phosphate buffer with Trehalose dihydrate 5.04% + Mannitol 1.31%, no Kolliphor P188 added;
Formulation B: 0.005mg/mL rhNGF in placebo + Phosphate buffer with Trehalose dihydrate 5.04% + Mannitol 1.31%+ 0.025% Kolliphor P188;
Formulation C: 0.005mg/mL rhNGF in placebo + Phosphate buffer with Trehalose dihydrate 5.04% + Mannitol 1.31%+ 0.05% Kolliphor P188;
Formulation D: 0.005mg/mL rhNGF in placebo + Phosphate buffer with Trehalose dihydrate 5.04% + Mannitol 1.31%+ 0.1% Kolliphor P188 (corresponding to the concentrations of rhNGF and Kolliphor P188 according to the reconstituted formulation of Example 1). Then, each sample was analysed by RP-HPLC.

RP-HPLC analysis was performed using Alliance® HPLC - e2695 quaternary system, using C4 - proteins analytical column, with a mobile phase consisting of a mixture of acetonitrile and water with trifluoracetic acid 0.05% (v/v) at a flow rate of 1.0 ml/min and injection volume of 50 μl. Detection was carried out at 220 nm in UV-DAD detector. Method applicability was tested for identification, assay and purity and impurities of rhNGF. rhNGF (recombinant human nerve growth factor) is supplied by Dompé farmaceutici S.p.a.

Subsequently, the recovery of rhNGF was determined as follows.

First of all, rhNGF concentration was calculated according to the following steps:

1. Integrate the peak of rhNGF in the chromatograms of Working Standard and Test Article Solutions.
2. Calculate the Response Factor to rhNGF for each injection of the working standard solution as follows:

$$FR = \frac{Aws \times Ds}{Cws}$$

where:

FR: Response factor

Aws: rhNGF peak area in the working standard
Ds: Working standard dilution (mL)
Cws: rhNGF Working Solution Concentration (mg/mL)

3.Calculate the rhNGF concentration (mg/mL) of Test Article as follows:

$$Conc.(mg/mL) = \frac{Ata}{MFR}$$

where: MRF: Mean Response Factor, Ata: Area of rhNGF in the Test Article Solutions.
4. Calculate the average value for the two Test Article Solutions and report as rhNGF concentration (mg/mL) of the rhNGF solution.

The HPLC results are summarized in the Tables below:

| **Formulation D** Kolliphor P188 0.1% (assumed as Reference) | Area rhNGF | | | | FR | Cone rhNGF | | | | Rec % vs Nominal | **Recovery % reference** |
|---|---|---|---|---|---|---|---|---|---|---|---|
| time | Areal | Area2 | Area3 | $Area_m$ | | Cone smp1 | Cone smp2 | Cone smp3 | Cone Av | | |
| 0 | 1840 16 | 1773 81 | 1743 22 | 178573 ,0 | 3,61E+ 07 | 0,0 | 4,9 | 4,8 | 4,9 | 98,9 | 100,0 |

| **Formulation A** no Kolliphor P188 | Area rhNGF | | | | FR | Cone rhNGF | | | | Rec % vs Nominal | **Recovery% Area A vs Area Ref** |
|---|---|---|---|---|---|---|---|---|---|---|---|
| time | Area1 | Area2 | Area3 | $Area_m$ | | Cone smp1 | Cone smp2 | Cone smp3 | Cone Av | | |
| 0 | 1024 54 | 1013 85 | 9275 2 | 98863, 7 | 2,06E+ 07 | 5,0 | 4,9 | 4,5 | 4,8 | 96,0 | 55,4 |

| **Formulation B** Kolliphor P188 0.025% | Area rhNGF | | | | FR | Cone rhNGF | | | | Rec % vs Nominal | **Recovery% Area B vs Area Ref** |
|---|---|---|---|---|---|---|---|---|---|---|---|
| time | Area1 | Area2 | Area3 | $Area_m$ | | Cone smp1 | Cone smp2 | Cone smp3 | Cone Av | | |
| 0 | 1651 29 | 1693 90 | 1686 01 | 167706 ,7 | 3,49E+ 07 | 4,7 | 4,9 | 4,8 | 4,8 | 96,2 | 93,9 |

| **Formulation C** Kolliphor P188 0.05% | Area rhNGF | | | | FR | Cone rhNGF | | | | Rec % vs Nominal | **Recovery% Area C vs Area Ref** |
|---|---|---|---|---|---|---|---|---|---|---|---|
| time | Area1 | Area2 | Area3 | $Area_m$ | | Cone smp1 | Cone smp2 | Cone smp3 | Cone Av | | |
| 0 | 1839 05 | 1792 11 | 1714 62 | 178192 ,7 | 3,74E+ 07 | 4,9 | 4,8 | 4,6 | 4,8 | 95,3 | 99,8 |

wherein "Nominal" indicates the nominal concentration of rhNGF, which is equal to 5.0.

To determine the recovery of rhNGF, Formulation with Kolliphor P188 0.1% (Formulation D) was assumed to be the Reference formulation.

All the formulations (Formulation A, B and C) have been compared with the Reference formulation.

The above results show that, without Kolliphor P188 (Formulation A), only half of the initial concentration of rhNGF remained in solution.

Instead, already at the minimum concentration tested (i.e. 0.025%), the Kolliphor P188 surfactant has been shown to guarantee a much higher recovery of rhNGF than that of Formulation A.

**Example 3 - Characterization of a pharmaceutical formulation in form of powder according to the present invention**

The freeze-dried and reconstituted pharmaceutical formulations obtained according to Example 1 were characterized at the temperature of 2-8°C, and at 25°C-60% UR, after 0, 1, 3, 6, 12, 18 and 24 months after lyophilization, according to the following Tables.

In particular, the measurements of the reconstituted formulation were carried out after reconstitution of a sample of the above-mentioned freeze-dried pharmaceutical formulation in 2mL WFI.

The method references used for determining the parameters reported in Tables below are according to GENERAL TESTS & ASSAYS from USP: Chap. 791 - pH; Chap. 785 - OSMOLALITY AND OSMOLARITY; Chap. 921 - WATER DETERMINATION (Karl Fischer).

| **2-8°C** | **0 months** | **1 month** | **3 months** | **6 months** | **12 months** | **18 months** | **24 months** |
|---|---|---|---|---|---|---|---|
| Appearance of lyophilizate | White Cake, free from visible foreign matter. | Compliant | Compliant | Compliant | Compliant | Compliant | Compliant |
| Appearance of reconstituted solution | Reconstituted solutions appear free from visible particulate; solutions are clear and colourless (comparable to WFI). | Compliant | Compliant | Compliant | Compliant | Compliant | Compliant |
| Reconstitution time (sec) | 21 | 18 | 10 | 8 | 9 | 13 | 10 |
| Osmolarity (mOsm/kg) | 68 | 69 | 69 | 70 | 74 | 57 | 69 |
| pH | 7,5 | 7,5 | 7,4 | 7,5 | 7,5 | 7,6 | 7,5 |
| Moisture content by KF (% w/w) | 0,6 | 0,6 | 0,8 | 0,6 | 1,1 | 1,0 | 1,2 |

| **25°C 60% UR** | **0 months** | **1 month** | **3 months** | **6 months** | **12 months** | **18 months** | **24 months** |
|---|---|---|---|---|---|---|---|
| Appearance of lyophilizate | White Cake, free from visible foreign matter. | Compliant | Compliant | Compliant | Compliant | Compliant | Compliant |

(continued)

| 25°C 60% UR | 0 months | 1 month | 3 months | 6 months | 12 months | 18 months | 24 months |
|---|---|---|---|---|---|---|---|
| Appearance of reconstituted solution | Reconstituted solutions appear free from visible particulate, solutions are clear and colourless (comparable to WFI). | Compliant | Compliant | Compliant | Compliant | Compliant | Compliant |
| Reconstitution Time (sec) | 21 | 18 | 9 | 9 | 9 | 13 | 12 |
| Osmolarity (mOsm/kg) | 68 | 69 | 69 | 70 | 74 | 60 | 69 |
| pH | 7,5 | 7,5 | 7,5 | 7,5 | 7,5 | 7,6 | 7,4 |
| Moisture content by KF (% w/w) | 0,6 | 0,8 | 1,1 | 1,3 | 1,9 | 2,5 | 2,9 |

As shown in the above Tables, the pharmaceutical formulation in form of powder, according to the present invention, is completely white and free of visible foreign matter.

No bilayer of the cake is observed at any tested point of time.

Moreover, the reconstituted formulations appear free from visible particulate; in particular, they appear clear and colourless, such as WFI, at any tested point of time.

Also, the other tested parameters are advantageously stable in time, in particular pH and osmolarity, showing the long-term stability of the pharmaceutical formulation in form of powder of the present invention.

**Example 4 - Comparative test.**

PEG 6000 is a typical stabilizing agent included in freeze-dried pharmaceutical formulations, because it contributes to stabilize proteins during freezing and thawing processes.

The inventors have carried out a comparative test between a rhNGF freeze-dried formulation containing PEG 6000, and a rhNGF freeze-dried formulation containing Kolliphor P188, to compare their protein stability during a freeze-drying process.

The following rhNGF liquid formulation containing PEG 6000, suitable for lyophilization, was prepared as follows.

Weight and solubilize in the appropriate volume of WFI the following excipients:

| Excipient | Concentration (mg/mL) | Concentration (%) |
|---|---|---|
| Sodium Phosphate dibasic anhydrous | 3.065 | 0.3065 |
| Sodium Phosphate monobasic monohydrate | 1.16 | 0.116 |
| PEG 6000 | 10.7 | 1.7 |
| Trehalose dihydrate | 50.40 | 5.04 |
| D-Mannitol | 13.10 | 1.31 |
| L-Methionine | 0.0107 | 0.001 |
| 1.0N Sodium Hydroxide | q.s. to pH 7.20 ± 0.20 | q.s. to pH 7.20 ± 0.20 |
| Water for Injection PhEur/USP WFI | q.b. | q.b. |

After the complete solubilization of excipients, rhNGF at the concentration of 0.020 mg/mL was added.

Separately, a rhNGF liquid formulation containing Kolliphor P188, suitable for lyophilization, was prepared

according to Example 1.

Then, both above liquid formulations were lyophilized according to the lyophilization process disclosed in Example 1.

The thus-obtained lyophilized samples were analysed by Differential Scanning Calorimetry (PerkinElmer DSC 8000) as follows:

- Heating from 10.00°C to 90.00°C at 20.00°C/min;
- Cooling from 90.00°C to 10.00°C at 20.00°C/min;
- Heating from 10.00°C to 200.00°C at 20.00°C/min.

The results are shown in thermograms reported as Figure 1 (rhNGF freeze dried formulation containing PEG 6000), and Figure 2 (rhNGF freeze dried formulation containing Kolliphor P188).

As shown in Figure 1, the endothermic peak of the thermogram shows an important crystallization of PEG 6000.

This physical phenomenon of crystallization indicates that PEG 6000 is a not a good protein stabilizer during the drying step of a lyophilization process, since the PEG crystallization determines the loss of stabilizing effect.

Furthermore, this effect has a negative effect on the consistency of the lyophilized cake despite the use of PEG 6000 as a bulking agent.

Thus, PEG 6000 has two unpredictable and surprising negative effects on a freeze-dried protein formulation, namely a negative effect on the protein stabilization during lyophilization drying steps, showing PEG as a worst lyoprotectant, and a negative effect on the morphology of the lyophilization cake, due PEG crystallization phenomena.

On the contrary, the thermogram reported in Figure 2, shows that Kolliphor P188 doesn't advantageously re-crystalize in formulation, so it doesn't cause any problem during drying steps of lyophilization.

Moreover, no negative effects on the morphology of the lyophilization cake are detected, when using Kolliphor P188 as surfactant in a protein formulation.

Therefore, Kolliphor P188 have proved to be a protein stabilizer better than PEG 6000, in particular in a rhNGF pharmaceutical formulation.

## Claims

1. A liquid pharmaceutical formulation, suitable for lyophilization, comprising:

   - nerve growth factor (NGF) at a concentration between 5 μg/ml and 300 μg/ml, preferably between 10 μg/ml and 50 μg/ml, more preferably between 15 μg/ml and 25 μg/ml;
   - at least one cryoprotectant agent at a concentration between 30 mg/ml and 60 mg/ml, preferably between 40 mg/ml and 55 mg/ml;
   - at least one bulking agent at a concentration between 5 mg/ml and 20 mg/ml, preferably between 10 mg/ml and 15 mg/ml;
   - at least one antioxidant at a concentration between 0.005 mg/ml and 0.020 mg/ml, preferably between 0.008 mg/ml and 0.015 mg/ml;
   - at least one poloxamer at a concentration of at least 0.25 mg/ml, preferably between 1 mg/ml and 4 mg/ml;
   - at least one buffer agent in an amount sufficient to maintain the pH of the formulation comprised between 6.5 to 8, and
   - water.

2. The liquid pharmaceutical formulation according to claim 1, wherein said nerve growth factor (NGF) is human nerve growth factor (hNGF), preferably having the amino acid sequence of SEQ. ID NO.1 or SEQ ID NO.2.

3. The liquid pharmaceutical formulation according to claim 1 or 2, wherein said at least one cryoprotectant agent is selected from fructose, glucose, mannose, sorbose, xylose, maltose, lactose, sucrose, dextran and trehalose or a mixture thereof, preferably trehalose.

4. The liquid pharmaceutical formulation according to any one of claims 1-3, wherein said at least one bulking agent is selected from mannitol, sorbitol, inositol, dulcitol, xylitol, arabitol, or a mixture thereof, preferably mannitol.

5. The liquid pharmaceutical formulation according to any one of claims 1-4, said at least one antioxidant is selected from L-methionine and cysteine, preferably L-methionine,

**6.** The liquid pharmaceutical formulation according to any one of claims 1-5, said at least one poloxamer is Poloxamer 188.

**7.** The liquid pharmaceutical formulation according to any one of claims 1-6, comprising:

- said nerve growth factor at a concentration between 10 μg/ml and 50 μg/ml, preferably between 15 μg/ml and 25 μg/ml;
- said trehalose at a concentration between 40 mg/ml and 55 mg/ml;
- said mannitol at a concentration between 10 mg/ml and 15 mg/ml;
- said L-methionine at a concentration between 0.008 mg/ml and 0.015 mg/ml;
- said Poloxamer 188 at a concentration between 1 mg/ml and 4 mg/ml;
- said buffer agent in an amount sufficient to maintain the pH of the formulation comprised between 6.5 to 8, and
- water.

**8.** A method for preparing a nerve growth factor (NGF) formulation in form of powder, comprising the step of: lyophilizing said liquid pharmaceutical formulation according to any one of claims 1-7, to obtain said nerve growth factor (NGF) formulation in form of powder.

**9.** A pharmaceutical formulation comprising nerve growth factor (NGF), in form of powder, obtained by said method according to claim 8.

**10.** The liquid pharmaceutical formulation, suitable for lyophilization, according to any one of claims 1-7, or said pharmaceutical formulation comprising nerve growth factor (NGF), in form of powder according to claim 9, for use in the treatment of a nerve injury.

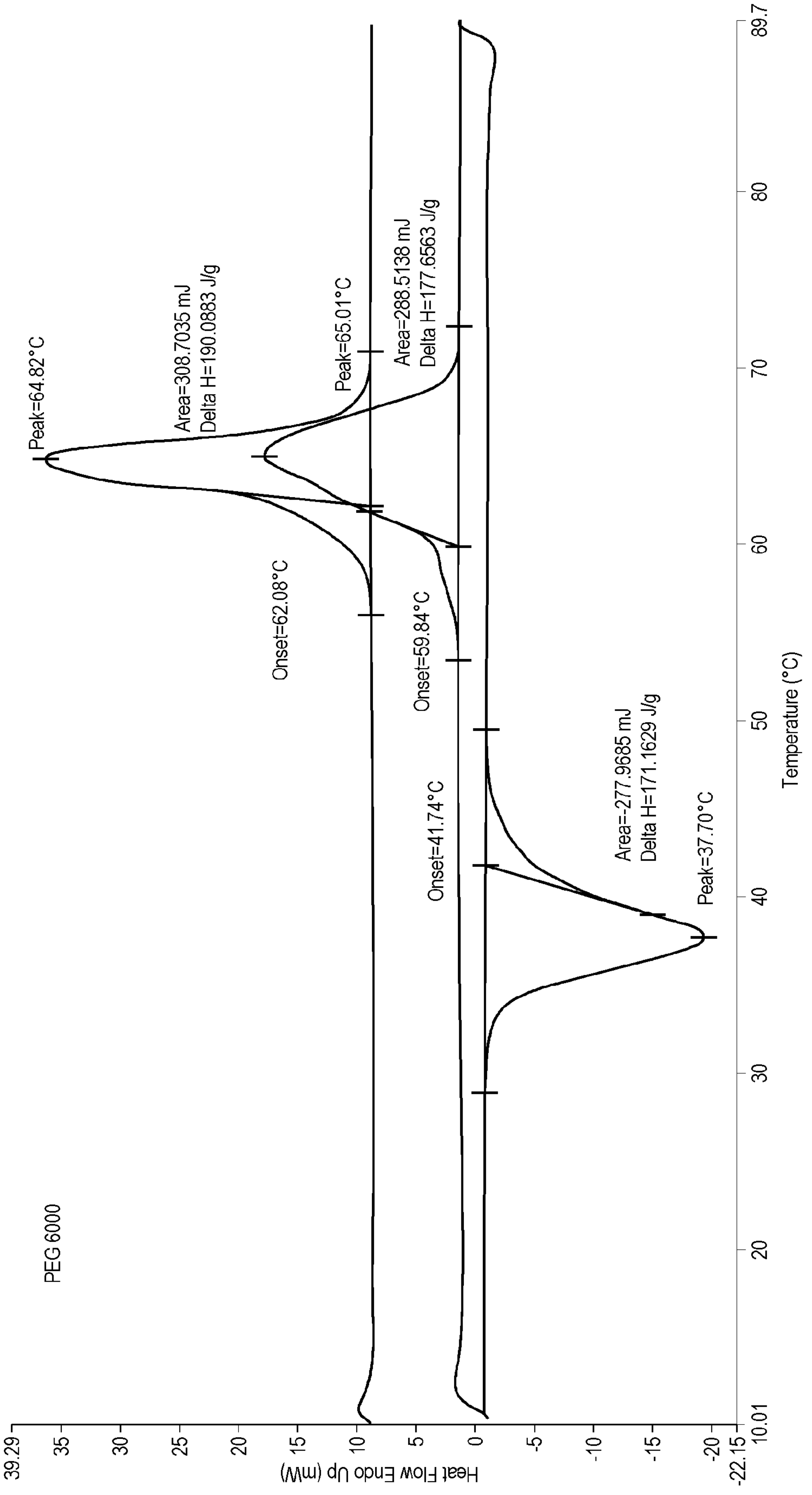
PEG 6000
Peak=64.82°C
Area=308.7035 mJ
Delta H=190.0883 J/g
Peak=65.01°C
Area=288.5138 mJ
Delta H=177.6563 J/g
Onset=62.08°C
Onset=59.84°C
Onset=41.74°C
Area=-277.9685 mJ
Delta H=171.1629 J/g
Peak=37.70°C
Heat Flow Endo Up (mW)
39.29
35
30
25
20
15
10
5
0
-5
-10
-15
-20
-22.15
10.01
20
30
40
50
60
70
80
89.7
Temperature (°C)

Figure 1

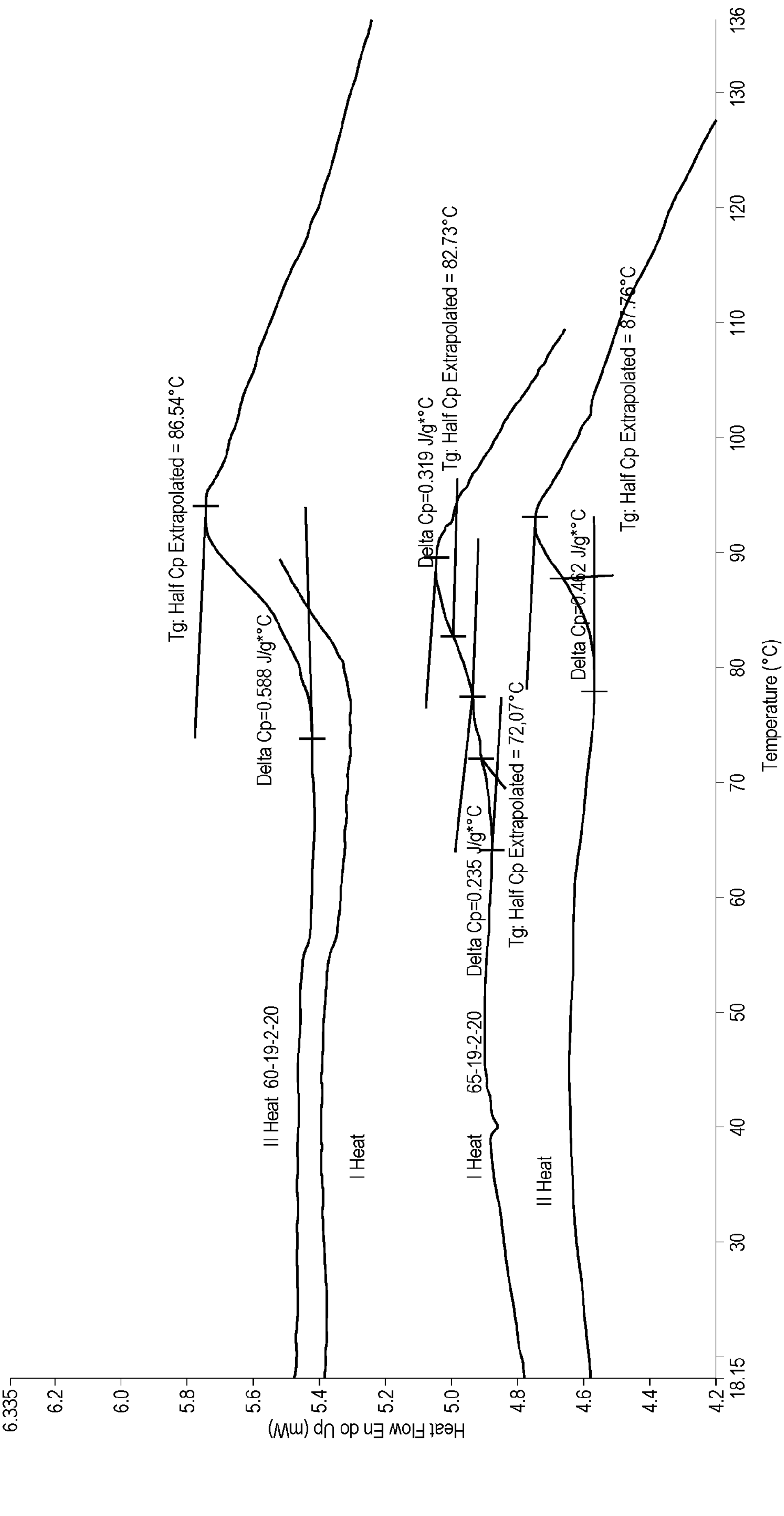
Heat Flow En do Up (mW)
6.335
6.2
6.0
5.8
5.6
5.4
5.2
5.0
4.8
4.6
4.4
4.2
18.15
30
40
50
60
70
80
90
100
110
120
130
136
Temperature (°C)
Tg: Half Cp Extrapolated = 86.54°C
Delta Cp=0.588 J/g*°C
II Heat 60-19-2-20
I Heat
Delta Cp=0.319 J/g*°C
Tg: Half Cp Extrapolated = 82.73°C
Delta Cp=0.235 J/g*°C
Tg: Half Cp Extrapolated = 72,07°C
65-19-2-20
I Heat
II Heat
Delta Cp=0.462 J/g*°C
Tg: Half Cp Extrapolated = 87.76°C

Figure 2

Europäisches Patentamt
European Patent Office
Office européen des brevets

# EUROPEAN SEARCH REPORT

Application Number
EP 23 18 8159

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | Anonymous: "ANNEX I - SUMMARY OF PRODUCT CHARACTERISTICS", London, 6 July 2017 (2017-07-06), pages 1-27, XP093128230, Retrieved from the Internet: URL:https://www.ema.europa.eu/en/documents/product-information/oxervate-epar-product-information_en.pdf [retrieved on 2024-02-06] * page 2 * * page 8 * ----- | 1-10 | INV. A61K9/00 A61K9/08 A61K38/18 A61K47/02 A61K47/18 A61K47/26 A61K47/34 |
| X | US 6 277 828 B1 (KNEPP VICTORIA M [US] ET AL) 21 August 2001 (2001-08-21) * claims 1-11 * * example 7 * * column 4, line 45 - column 7, line 65 * ----- | 1-10 | |
| A | US 2003/203040 A1 (CLELAND JEFFREY L [US] ET AL) 30 October 2003 (2003-10-30) * paragraph [0095] - paragraph [0139] * * claims 1-28 * ----- | 1-10 | TECHNICAL FIELDS SEARCHED (IPC) A61K |

The present search report has been drawn up for all claims

2

| Place of search | Date of completion of the search | Examiner |
|---|---|---|
| Munich | 4 March 2024 | Schifferer, Hermann |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 23 18 8159

This annex lists the patent family members relating to the patent documents cited in the above-mentioned European search report. The members are as contained in the European Patent Office EDP file on
The European Patent Office is in no way liable for these particulars which are merely given for the purpose of information.

04-03-2024

| Patent document cited in search report | Publication date | Patent family member(s) | Publication date |
|---|---|---|---|
| US 6277828 B1 | 21-08-2001 | AT E226085 T1 | 15-11-2002 |
| | | AU 677699 B2 | 01-05-1997 |
| | | BG 62951 B1 | 29-12-2000 |
| | | BR 9407278 A | 01-10-1996 |
| | | CA 2169834 A1 | 02-03-1995 |
| | | CN 1133012 A | 09-10-1996 |
| | | CZ 292422 B6 | 17-09-2003 |
| | | DE 69431562 T2 | 18-06-2003 |
| | | DK 0721343 T3 | 17-02-2003 |
| | | EP 0721343 A1 | 17-07-1996 |
| | | ES 2181723 T3 | 01-03-2003 |
| | | FI 960750 A | 20-02-1996 |
| | | HK 1012990 A1 | 13-08-1999 |
| | | HU 228152 B1 | 28-12-2012 |
| | | IL 110725 A | 27-09-2004 |
| | | IL 124941 A | 10-12-2006 |
| | | JP 4592830 B2 | 08-12-2010 |
| | | JP H10508000 A | 04-08-1998 |
| | | JP 2007314572 A | 06-12-2007 |
| | | KR 100341193 B1 | 21-06-2002 |
| | | LT 4051 B | 25-10-1996 |
| | | LV 11279 A | 20-06-1996 |
| | | NO 317627 B1 | 29-11-2004 |
| | | NZ 271873 A | 28-10-1996 |
| | | PL 313084 A1 | 27-05-1996 |
| | | PT 721343 E | 31-01-2003 |
| | | RO 114742 B1 | 30-07-1999 |
| | | RU 2126265 C1 | 20-02-1999 |
| | | SI 9420048 A | 31-10-1996 |
| | | SK 18396 A3 | 02-10-1996 |
| | | TW 427905 B | 01-04-2001 |
| | | UA 43348 C2 | 17-12-2001 |
| | | US 6277828 B1 | 21-08-2001 |
| | | US 2001007662 A1 | 12-07-2001 |
| | | WO 9505845 A1 | 02-03-1995 |
| | | ZA 946333 B | 19-02-1996 |
| US 2003203040 A1 | 30-10-2003 | US 2002004481 A1 | 10-01-2002 |
| | | US 2003203040 A1 | 30-10-2003 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 0022119 A1
- WO 2013092776 A1

### Non-patent literature cited in the description


- **THAKRAL S. et al.** Stabilizers and their interaction with formulation components in frozen and freeze-dried protein formulations. *Advanced Drug Delivery Reviews*, 2021, vol. 173, 1-19